(19) Europäisches Patentamt
European Patent Office
Office européen des brevets

(11) Veröffentlichungsnummer: 0 252 482
A2

# (12) EUROPÄISCHE PATENTANMELDUNG

(21) Anmeldenummer: 87109750.7

(22) Anmeldetag: 07.07.87

(51) Int.Cl.³: C 07 D 311/92
C 07 D 405/12, A 61 K 31/35

(30) Priorität: 11.07.86 DE 3623300

(43) Veröffentlichungstag der Anmeldung:
13.01.88 Patentblatt 88/2

(84) Benannte Vertragsstaaten:
AT BE CH DE ES FR GB GR IT LI LU NL SE

(71) Anmelder: HOECHST AKTIENGESELLSCHAFT
Postfach 80 03 20
D-6230 Frankfurt am Main 80(DE)

(72) Erfinder: Khandelwal, Yatendra, Dr.
M-8, Hoechst Executive Quarters Darga Road
Mulund West Bombay 400 082(IN)

(72) Erfinder: Rajgopalan, Ramanujam
No. 205, B Wing, Aarthi Sarojini Naidu Road
Mulund West Bombay 400 080(IN)

(72) Erfinder: Dohadwalla, Alihussein Nomanbhai, Dr.
Noman Mansion, 139 C
Cumballa Hill Bombay 400 036(IN)

(72) Erfinder: Rupp, Richard Helmut, Dr.
"Niladri" 66 Nepan Sea Road
Bombay 400 006(IN)

(72) Erfinder: De Souza, Noel John, Dr.
Melrose 62 Pali Hill
Bandra Bombay 400 050(IN)

(54) 7-Acyloxy-6-aminoacyloxypolyoxylabdane, Verfahren zu ihrer Herstellung und ihre Verwendung als Arzneimittel.

(57) Die vorliegende Erfindung betrifft 7-Acyloxy-6-aminoacyloxypolyoxylabdane und ihre physiologisch unbedenklichen Salze sowie ein Verfahren zu ihrer Herstellung. Die erfindungsgemäßen Verbindungen weisen die der Klasse der Polyoxylabdane zugehörigen pharmakologischen Eigenschaften auf, zeigen aber insbesondere eine ausgeprägte selektive blutdrucksenkende Wirkung.

EP 0 252 482 A2

Croydon Printing Company Ltd

HOECHST AKTIENGESELLSCHAFT HOE 86/F 153

Beschreibung

7-Acyloxy-6-aminoacyloxypolyoxylabdane, Verfahren zu ihrer Herstellung und ihre Verwendung als Arzneimittel

Die vorliegende Erfindung betrifft 7-Acyloxy-6-aminoacyloxypolyoxylabdane und ihre physiologisch unbedenklichen Salze, ein Verfahren zu ihrer Herstellung und ihre Verwendung als Arzneimittel.

Polyoxylabdane und deren Derivate sind bereits beschrieben worden in: Deutsche Offenlegungsschriften Nr. 25 57 784, 26 40 275 und 26 54 796; Tetrahedron Letters Nr. 19, S. 1669 - 1672 (1977); J. Chem. Soc., Perkin Trans. 1, 767 (1982) sowie in den Europäischen Patentanmeldungen EP-A-0 217 372, EP-A-0 191 166 und EP-A-0 193 132.

Auf Grund der pharmakologischen Eigenschaften der im obigen Stand der Technik beschriebenen Polyoxylabdane und deren Derivate sind diese zur Verwendung bei der Behandlung von Herz- und Kreislaufkrankheiten, hohem Blutdruck, Glaukom, Allergien, Bronchialasthma sowie als Immunomodulatoren geeignet.

Die erfindungsgemäßen Polyoxylabdanderivate sind weder in den zum Stand der Technik genannten Veröffentlichungen beschrieben noch durch diese nahegelegt. Mit den erfindungsgemäßen Verbindungen zum Teil strukturell verwandte Verbindungen des Standes der Technik sind die Derivate, die eine 6-Aminoacyloxygruppe tragen.

Der wesentliche Unterschied zwischen den erfindungsgemäßen Verbindungen und denen des Standes der Technik liegt darin, daß in den erfindungsgemäßen Verbindungen neben einer Aminoacyloxygruppe in 6-Stellung der Substituent in 7-Stellung eine Acyloxygruppe ist, während in den Verbindungen des Standes der Technik neben einer Aminoacyloxygruppe in 6-Stellung der Substituent in

7-Stellung entweder nur eine Hydroxyl- oder eine Aminoacyloxygruppe ist. Überraschenderweise verändert diese Strukturänderung das pharmakologische Profil der Verbindungen, wodurch sie sich insbesondere zur Behandlung hohen Blutdrucks besser eignen als andere Verbindungen des Standes der Technik, die gleichzeitig auch andere für Polyoxylabdan-Derviate typische pharmakologische Wirkungen zeigen.

Diese und weitere Aufgaben sind aus der nachfolgenden ausführlichen Beschreibung ersichtlich.

Gegenstand vorliegender Erfindung sind neue 7-Acyloxy-6-aminoacyloxyderivate von Polyoxylabdanen und Verfahren zu deren Herstellung. Die erfindungsgemäßen neuen 7-Acyloxy-6-aminoacyloxyderivate von Polyoxylabdanen und deren pharmazeutisch unbedenkliche Salze besitzen wertvolle, wie im Stand der Technik beschriebene pharmakologische Eigenschaften, zeigen aber insbesondere blutdrucksenkende Eigenschaften.

Erfindungsgemäß entsprechen die neuen Polyoxylabdanderivate der Formel I

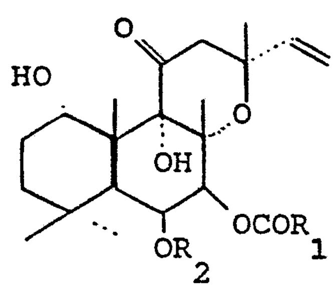

I

worin $R_1$ für geradkettige oder verzweigte, gegebenenfalls einfach oder mehrfach durch Hydroxyl- oder Halogengruppen substituierte $C_1$-$C_{20}$-Alkylgruppen und $R_2$ für die Gruppe

Als substituierte Alkylgruppen eignen sich für $R_5$ und $R_6$ z.B. Hydroxyalkyl wie Hydroxyäthyl, Carboxyalkyl wie Carboxyäthyl, Carbalkoxyalkyl wie Carbäthoxyäthyl oder eine Äthylindolgruppe.

Als Cycloalkylgruppen kommen solche mit 3 bis 6 C-Atomen infrage, insbesondere die Cyclohexylgruppe.

Geeignete Beispiele für Aralkylgruppen sind Phenyl-$C_1$-$C_4$-alkylgruppen, beispielsweise eine Benzylgruppe, in der die Phenylgruppe einfach oder mehrfach durch Substituenten wie Halogen, $C_1$-$C_4$-Alkyl, $C_1$-$C_4$-Alkoxy, Nitro oder Trifluormethylgruppen substituiert sein kann.

Als Arylgruppen für die Substituenten $C_3$-$C_6$-eignen sich Phenylgruppen, die einfach oder mehrfach durch Substituenten wie Halogen-, $C_1$-$C_4$-Alkyl-, $C_1$-$C_4$-Alkoxy-, Nitro- oder Trifluormethylgruppen substituiert sein können.

Geeignete Beispiele für Heterocyclen als Definition von $R_6$ sind Chinolin und Pyridin.

Alkanoylgruppen sind beispielsweise Formyl-, Acetyl-, Propionyl-, Butyryl-, Isobutyryl-, Valeryl-, Palmityl- und Bromisobutyrylgruppen. Die Alkanoylgruppen können eine oder mehrere Doppelbindungen, wie beispielsweise als Acrylyl-, Stearyl- oder Oleoylgruppen, eine oder mehrere Dreifachbindungen und zusätzlich eine oder mehrere Doppelbindungen enthalten. Ein Beispiel für solche Alkinylgruppen istdie Propiolylgruppe. Ein geeigneter Vertreter für Aroylgruppen ist die Benzoylgruppe, in der die Phenylgruppe durch ein oder mehrere Substituenten wie $C_1$-$C_4$-Alkyl-, $C_1$-$C_4$-Alkoxy-, Halogen-, Nitro- oder Trifluormethylgruppen substituiert sein kann. Beispiele für Aralkanoyl- bzw. Heteroaroylgruppen sind Phenylacetyl- bzw. Pyridin-3-carbonylgruppen.

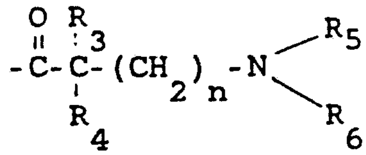

stehen, wobei $R_3$ und $R_4$, die gleich oder verschieden sein können, für Wasserstoff oder eine Alkyl- oder Arylgruppe, n für eine ganze Zahl von 0 bis 10 und $R_5$, falls $R_6$ Wasserstoff bedeutet, für Wasserstoff, Alkyl, substituiertes Alkyl, Cycloalkyl, Aralkyl, Aryl, einen Heterocyclus, Amino, substituiertes Amino, Hydroxyl, Acyl, Dialkylaminoalkyl, Carbamyl, Carboxyalkyl oder Carbalkoxyalkyl stehen, oder, falls sie gleich sind, $R_5$ und $R_6$ für Alkyl, substituiertes Alkyl, Aryl oder Aralkyl stehen, oder, falls $R_5$ eine Alkylgruppe bedeutet, $R_6$ für eine substituierte Alkyl-, Cycloalkyl-, Aralkyl- oder Dialkylaminoalkylgruppe steht, oder $R_5$ und $R_6$ zusammen mit dem Stickstoffatom, an das sie gebunden sind, für einen Heterocyclus stehen, der zusätzlich zum N-Atom ein oder mehrere Heteroatome enthalten kann und gegebenenfalls an einer oder mehreren Stellen mit Alkyl, Aralkyl, Hydroxyalkyl, Aryl, Hydroxyl oder weiteren heterocyclischen Gruppen substituiert ist, sowie deren physiologisch unbedenkliche Salze.

Die obige Definition der neuen 7-Acyloxy-6-aminoacyloxyderivate von Polyoxylabdanen schließt sämtliche möglichen Stereoisomeren und deren Gemische ein.

Für die Substituenten $R_1$-$R_6$ geeignete Alkylgruppen sind beispielsweise geradkettige oder verzweigte Reste mit bis zu 20, vorzugsweise bis zu 6, insbesondere bis zu 4 Kohlenstoffatomen, wie z.B. Methyl-, Äthyl-, Isopropyl-, t-Butyl- und n-Butylgruppen. Geeignete Beispiele für R = substituiertes Alkyl sind Hydroxymethyl-, 2,3-Dihydroxypropyl- und Chlormethylgruppen.

Geeignete Dialkylaminoalkylgruppen ist Di-($C_1$-$C_4$-alkyl)-amino-$C_1$-$C_4$-alkyl, z.B. die Diäthylaminoäthylgruppe.

Geeignete heterocyclische Gruppen, die durch $R_5$ und $R_6$ zusammen mit dem Stickstoffatom, an das sie gebunden sind, gebildet werden, sind beispielsweise die Piperidino-, Pyrrolidino-, Morpholino-, Piperazino-, Thiomorpholino-, Imidazolino- und Theophyllin-Reste , die gegebenenfalls ein oder mehrfach durch $C_1$-$C_4$-Alkyl-, $C_1$-$C_4$-Alkoxy-, Aryl-, Aryl-$C_1$-$C_4$-aralkyl, Hydroxyl-, -Amino oder substituiertes $C_1$-$C_4$-Alkyl-, Aryl- oder Aminogruppen, z. B. die Benzoylaminogruppe, substituiert sein können.

n ist bevorzugt eine ganze Zahl von 0 bis 5.

Beispiele für geeignete Salze der erfindungsgemäßen Verbindungen mit anorganischen oder organischen Säuren sind das Hydrochlorid, Hydrobromid, Sulfat, Phosphat, Acetat, Oxalat, Tartrat, Citrat, Maleinat oder Fumarat.

Für den Substituenten $R_1$ sind bevorzugt Methyl, Hydroxymethyl oder Halogenmethylgruppen. Für den Rest

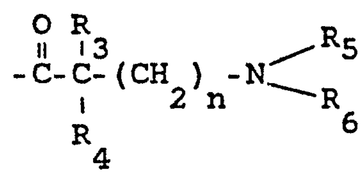

kommen z.B. die unten aufgeführten infrage:

N-Äthylaminoacetyl,
N,N-Diäthylaminoacetyl,
N-Benzyl-N-methylaminoacetyl,
N-Äthyl-N-methylaminoacetyl,
4'-Phenylpiperidinoacetyl,
Anilinoacetyl,
N-Methylanilinoacetyl,
N,N-Diphenylaminoacetyl,
ß-Phenyläthylaminoacetyl,

ß-Phenyläthylaminoacetyl,
N-Acetylaminoacetyl,
N-Phenacylaminoacetyl,
4'-Hydroxypiperidinoacetyl,
Thiomorpholinoacetyl,
iso-Propylaminoacetyl,
t-Butylaminoacetyl,
Diäthanolaminoacetyl,
N-(N',N'-Diäthylaminoäthyl)-N-methylaminoacetyl,
Hydrazinoacetyl,
Hydroxyaminoacetyl,
Phenylhydrazinoacetyl,
Aminoacetyl,
Benzylaminoacetyl,
N-Benzyl-N-ß-äthanolaminoacetyl,
4-Benzoylaminopiperidinoacetyl,
4-Hydroxy-4-phenylpiperidonoacetyl,
ß-Alaninoacetyl,
t-Butyl-ß-alaninoacetyl,
Tryptaminoacetyl,
Aminochinolinoacetyl,
Imdidazolylacetyl,
Theophyllinoacetyl,
ß-(N-Äthylamino)-propionyl,
ß-(N-Benzyl-N-methylamino)-propionyl,
ß-(N,N-Diäthylamino)-propionyl,
ß-(4'-Phenylpiperidino)-propionyl,
ß-(Anilino)-propionyl,
ß-(N-Methylanilino)-propionyl,
ß-(N,N-Diphenylamino)-propionyl,
ß-(ß'-Phenyläthylamino)-propionyl,
ß-(N-Acetylamino)-propionyl,
ß-(N-Phenacylamino)-propionyl,
ß-(4'-Hydroxypiperidino)-propionyl,
ß-(Thiomorpholino)-propionyl,
ß-(Isopropylamino)-propionyl,

ß-(t-Butylamino)-propionyl,
ß-(Diäthanolamino)-propionyl,
ß-N-(N',N'-Diäthylaminoäthyl)-N-methylaminopropionyl,
ß-Hydrazinopropionyl,
ß-Hydroxylaminopropionyl,
ß-Phenylhydrazinopropionyl,
ß-Aminopropionyl,
ß-Benzylaminopropionyl,
ß-(N-Benzyl-N-ß-äthanolamino)-propionyl,
ß-(N-Benzoylaminopiperidino)-propionyl,
ß-(4-Hydroxy-4-phenylpiperidino)-propionyl,
ß-(ß'-Alanino)-propionyl,
ß-(t-Butyl-ß'-alanino)-propionyl,
ß-Tryptaminopropionyl,
ß-Aminochinolinopropionyl
ß-Imidazolinopropionyl,
ß-(Theophyllino)-propionyl,
ß-(N-Äthyl-N-methylamino)-propionyl,
α-(N-Äthylamino)-propinyl,
α-(N,N-Diäthylamino)-propionyl,
α-(N-Benzyl-N-methylamino)-propionyl,
α-(N-Äthyl-N-methylamino)-propionyl,
α-(4'-Phenylpiperidino)-propionyl,
α-(Anilino)-propionyl,
α-(N-Methylanilino)-propionyl,
α-(N,N-Diphenylamino)-propionyl,
α-(ß'-Phenyläthylamino)-propionyl,
α-(N-Acetylamino)-propionyl,
α-(N-Phenylacylamino)-propionyl,
α-(4'Hydroxypiperidino)-propionyl,
α-(Thiomorpholino)-propionyl,
α-(Isopropylamino)-propionyl,
α-(t-Butylamino)-propionyl,
α-(Diäthanolamino)-propionyl,
α-[N-(N',N'-Diäthylaminoäthyl)-N-methylamino]-propionyl,
α-(Hydrazino)-propionyl,

α-(Hydroxylamino)-propionyl,
α-(Phenylhydrazino)-propionyl,
α-(Amino)-propionyl,
α-(Benzylamino)-propionyl,
α-(N-Benzyl-N-ß-äthanolamino)-propionyl,
α-(N-Benzoylaminopiperidino)-propionyl,
α-(4-Hydroxy-5-phenylpiperidino)-propionyl,
α-(ß-Alanino)-propionyl,
α-(t-Butylalanino)-propionyl,
α-(Tryptamino)-propionyl,
α-(Aminoquinolino)-propionyl,
α-Imidazolopropionyl,
α-Theophyllinopropionyl,
γ-(N-Äthylamino)-butyryl,
γ-(N,N-Diäthylamino)-butyryl,
γ-(N-Benzyl-N-methylamino)-butyryl,
γ-(N-Äthyl-N-methylamino)-butyryl,
γ-4'-Phenylpiperidino)-butyryl,
γ-Anilinobutyryl,
γ-(N-Methylanilino)-butyryl,
γ-(N,N-Diphenylamino)-butyryl,
γ-ß'-Phenyläthylamino)-butyryl,
γ-(N-Acetylamino)-butyryl,
γ-(N-Phenacylamino)-butyryl,
γ-(4'-Hydroxypiperidino)-butyryl,
γ-(Thiomorpholino)-butyryl,
γ-(Isopropylamino)-butyryl,
γ-(t-Butylamino)-butyryl,
γ-(Diäthanolamino)-butyryl,
γ-[N-(N',N'-Diäthylaminoäthyl)-N-methylamino]-butyryl,
γ-Hydrazinobutyryl,
γ-Hydroxylaminobutyryl,
γ-Phenylhydrazinobutyryl,
γ-(Amino)-butyryl,
γ-(Benzylamino)-butyryl,
γ-(N-Benzyl-N-ß-äthanolamino)-butyryl,

γ-(4-Benzylaminopiperidino)-butyryl,
γ-(4-Hydroxy-4-phenylpiperidino)-butyryl,
γ-(ß'-Alanino)-butyryl,
γ-(t-Butyl-ß'-alanin)-acetylbutyryl,
γ-(Tryptamino)-butyryl,
γ-(Aminochinolino)-butyryl,
γ-(Imidazo)-butyryl,
γ-(Theophyllino)-butyryl,
δ-(N-Äthylamino)-valeryl,
δ-(N,N-Diäthylamino)-valeryl,
δ-(N-Benzyl-N-methylamino)-valeryl,
δ-(N-Äthyl-N-methylamino)-valeryl,
δ-(4'-Phenylpiperidino)-valeryl,
δ-(Anilino)-valeryl,
δ-(N-Methylanilino)-valeryl,
δ-(N,N-Diphenylamino)-valeryl,
δ-(ß-Phenyläthylamino)-valeryl,
δ-(N-Acetylamino)-valeryl,
δ-(N-Phenacylamino)-valeryl,
δ-(4'-Hydroxypiperidino)-valeryl,
δ-(Thiomorpholino)-valeryl,
δ-(Isopropylamino)-valeryl,
δ-(t-Butylamino)-valeryl,
δ-(Diäthanolamino)-valeryl,
δ-[N-(N',N'-Diäthylaminoäthyl)-N-methylamino]-valeryl,
δ-(Hydrazino)-valeryl,
δ-(Amino)-valeryl,
δ-(Benzylamino)-valeryl,
δ-(Phenylhydrazino)-valeryl,
δ-(N-Benzyl-N-ß-äthanolamino)-valeryl,
δ-(4-Benzoylaminopiperidino)-valeryl,
δ-(4-Hydroxy-4-phenylpiperidino)-valeryl,
δ-(ß-Alanino)-valeryl,
δ-(t-Butyl-ß-alanino)-valeryl,
δ-(Tryptammino)-valeryl,
δ-(Aminochinolino)-valeryl,

δ-(Imidazo)-valeryl,
δ-(Theophyllino)-valeryl,
12-Piperidinolauroyl,
12-Morpholinolauroyl,
12-Pyrrolidinolauroyl,
12-(4'-Methylpiperazino)-lauroyl.

Die folgenden erfindungsgemäßen Verbindungen sind bevorzugte Beispiele:

7β-Acetoxy-6β-[α'-(N,N-dimethylamino)propionyloxy]-1α,9α-dihydroxy-8,13-epoxy labd-14-en-11-on hydroxychlorid (I-Isomer).

7β-Acetoxy-6β-[α'-(N,N-dimethylamino)propionyloxy]-1α,9α-dihydroxy-8,13-epoxy labd-14-en-11-on hydrochlorid hemihydrat (Il-Isomer).

7β-Acetoxy-1α,9α-dihydroxy-8,13-epoxy-6β-pyrrolidino-acetoxy labd-14-en-11-on hydrochlorid.

7β-Acetoxy-6β-[4'-benzoylaminopiperidino acetoxy]-1α,9α-dihydroxy-8,13-epoxy-labd-14-en-11-on hydrochlorid dihydrat.

7β-Acetoxy-1α,9α-dihydroxy-8,13-epoxy-6β-morpholino acetoxy labd-14-en-11-on hydrochlorid hydrat.

7β-Acetoxy-1α,9α-dihydroxy-8,13-epoxy-6β-(N-methyl piperazino acetoxy)labd-14-en-11-on dihydrochlorid hydrat.

1α,9α-Dihydroxy-8,13-epoxy-6β-piperidino acetoxy-7β-propionyloxy-labd-14-en-11-on hydrochlorid.

7β-Acetoxy-1α,9α-dihydroxy-8,13-epoxy-6β [α'-piperidino propionyloxy]-labd-14-en-11-on hydrochlorid (I-Isomer).

7β-Acetoxy-1α,9α-dihydroxy-8,13-epoxy-6β-[α'-piperidino propionyloxy]-labd-14-en-11-on hydrochlorid (II-Isomer).

7β-Acetoxy-1α,9α-dihydroxy-8,13-epoxy-6β-[α'-morpholino propionyloxy]-labd-14-en-11-on-hydrochlorid monohydrat (I-Isomer).

7β-Acetoxy-1α,9α-dihydroxy-8,13-epoxy-6β-[α'-morpholino propionyloxy]-labd-14-en-11-on-hydrochlorid trihydrat (II-Isomer).

7β-Acetoxy-1α,9α-dihydroxy-8,13-epoxy-6β-[α'-piperidino propionyloxy]-labd-14-en-11-on hydrochlorid.

7β-Acetoxy-1α,9α-dihydroxy-8,13-epoxy-6β-[α'-morpholino propionyloxy]-labd-14-en-11-on hydrochlorid.

7β-Acetoxy-1α,9α-dihydroxy-8,13-epoxy-6β-[α'-piperidino butyryloxy]-labd-14-en-11-on hydrochlorid hydrat.

Gegenstand der Erfindung ist ferner ein Verfahren zur Herstellung der neuen 7-Acyloxy-6-aminoacyloxy-polyoxylabdane der Formel I. Das Verfahren ist dadurch gekennzeichnet, daß man der Formel II entsprechende Verbindungen (siehe das folgende Schema)

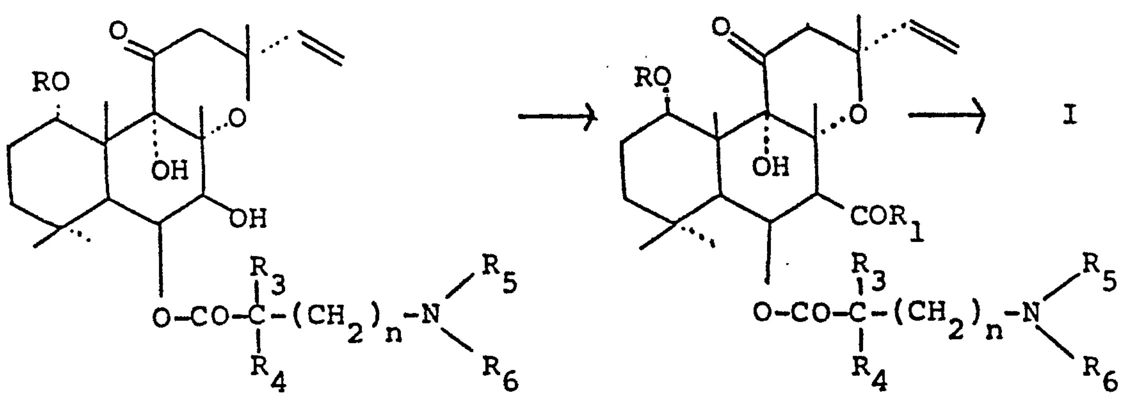

II III

worin R eine zum Schutz einer Hydroxylgruppe verwendete Gruppe, z.B. eine Äther-, Acetal-, Ketal- oder Estergruppe,

bevorzugt Trimethylsilyl, Methoxyäthoxymethyl oder Methoxymethyl bedeutet und die Substituenten $R_3$ bis $R_6$ die oben angegebenen Bedeutungen haben, nach üblichen Methoden zu einer Verbindung der Formel III acyliert. Beispielsweise kann die Verbindung der Formel II in üblicher Weise mit entsprechenden Carbonsäureanhydriden oder Carbonsäurechloriden der Formeln $R_1COOCOR_1$ bzw. $R_1COCl$ umgesetzt werden.

In einer bevorzugten Ausführungsform wird eine Verbindung der Formel II in einem wasserfreien organischen Lösungsmittel wie Essigester bei Temperaturen im Bereich von 10 bis 40°C, bevorzugt 20 bis 30°C mit einem Gemisch aus Essigsäure, Dicyclohexylcarbodiimid und 4-Dimethylaminopyridin umgesetzt. Das Reaktionsprodukt der Formel III, worin R und $R_3$ bis $R_6$ die oben angegebenen Bedeutungen haben, wird durch Behandlung des Reaktionsgemischs mit überschüssiger Essigsäure, Abtrennung der Essigesterschicht, Waschen mit Wasser und gesättigter Natriumcarbonatlösung, Trocknen über wasserfreiem Natriumsulfat und Einengen der Essigesterschicht isoliert.

Die Schutzgruppe in 1-Stellung kann nach üblichen Methoden abgespalten werden. So behandelt man z.B. Verbindungen der Formel III, worin R für eine t-Butyl-dimethylsilylgruppe steht, mit Reagenzien wie Tetrabutylammoniumfluorid bei einer Temperatur im Bereich von 20 bis 30°C, um die Hydroxylgruppe in 1-Stellung freizumachen und die gewünschten Verbindungen der Formel I zu erhalten. Steht R für eine Methoxymethylschutzgruppe in 1-Stellung in Verbindungen der Formel III, so erfolgt die Freisetzung durch Behandlung mit para-Toluolsulfonsäure, um die gewünschten Verbindungen der Formel I zu erhalten. Solche Methoden zur Entfernung von an OH-Gruppen gebundenen Schutzgruppen sind in der Literatur hinlänglich beschrieben und dem Fachmann bekannt (siehe z.B. "Protective Groups

in Organic Chemistry" Chapter 3, Plenum Press, London, New York 1973, S. 95 ff).

Verbindungen der Formel II werden nach in der EP-A-0 217 372 beschriebenen Methoden aus einem Zwischenprodukt der Formel IV

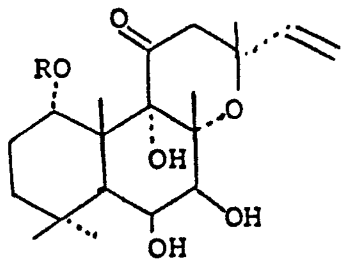

IV

hergestellt, worin R dieselbe Bedeutung wie in Formel II hat. Verbindungen der Formel IV stellt man aus Forskolin (7ß-Acetoxy-8,13-epoxy-aα,9α-trihydroxy-labd-14-en-11-on) gemäß folgender Reaktionsfolge her:

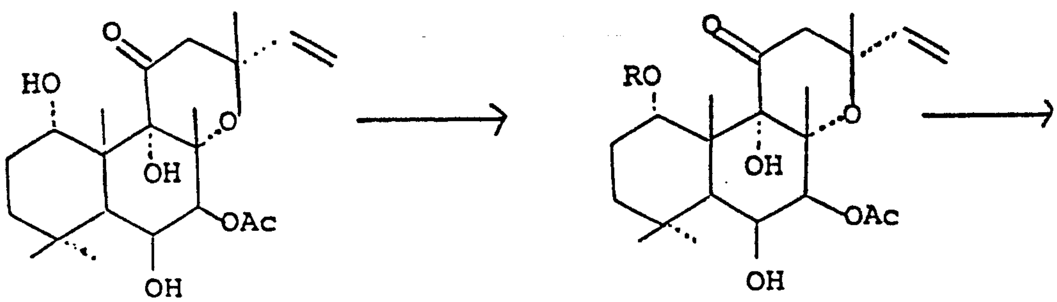

IV

V VI

wobei die 1-OH-Gruppe des Forskolins (V) nach dem Fachmann bekannten Methoden (siehe Reagents for Org. Synthesis [Reagenzien zur organischen Synthese], L.F. Fieser und M. Fieser, John Wiley and Sons, Inc., Band 1 bis 11) mit einer wie oben definierten Gruppe R geschützt ist. Die Acetylgruppe in 7-Stellung von Verbindungen der Formel VI,

worin R die oben angegebene Bedeutung hat, wird anschließend durch alkalische Hydrolyse ebenfalls nach dem Fachmann bekannten Methoden [siehe J.C.S. Perkin I, 796 (1982)] abgespalten. Derartige Methoden sind auch in den hierin beschriebenen Beispielen erläutert.

Die erfindungsgemäßen Verbindungen und ihre Salze weisen die der Klasse der Polyoxylabdane und ihrer Derivate, zugehörigen pharmakologischen Eigenschaften auf, zeigen aber insbesondere noch eine ausgeprägte selektive blutdrucksenkende Wirkung. Diese Eigenschaften werden durch die nachfolgend wiedergegebenen pharmakologischen Untersuchungen, die zur Bewertung der erfindungsgemäßen Verbindungen und deren Salzen durchgeführt wurden, belegt. Die erhaltenen Ergebnisse finden sich in den beigefügten Tabellen.

1. Blutdruck an der Katze:
Katzen beiderlei Geschlechts von 3 bis 4 kg Gewicht wurden mit Äther narkotisiert und unter Chloralose-Narkose (70 mg/kg, i.v.) gehalten. Sowohl die Oberschenkelarterie als auch -vene wurden zur Aufzeichnung des Blutdrucks und zur Verabreichung von Medikamenten kanüliert. Der Blutdruck in der Oberschenkelarterie wurde über einen Messwandler Statham 23 Db auf einem physiologischen Nihon-Kohden-Registriergerät aufgezeichnet. Die zu prüfende Verbindung wurde in destilliertem Wasser gelöst und intravenös verabreicht, und der Blutdruckabfall und die Dauer der blutdrucksenkenden Wirkung wurden notiert.

Die mit diesem Modell für typische erfindungsgemäße Verbindungen erhaltenen Resultate sind in der nachfolgenden Tabelle angegeben:

| Verbindung | | | Dosis (mg/kg) | Blutdruck-abfall (mm Hg) | Dauer (Min.) |
|---|---|---|---|---|---|
| 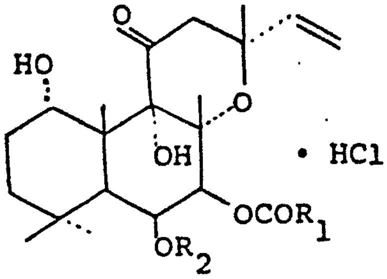 • HCl | | | | | |
| $R_1$ | | $R_2$ | | | |
| $CH_3$ | Isomer I | $COCH\ NMe_2$<br>$CH_3$ | 0.1 | 30 | 120 |
| $CH_3$ | Isomer II | $COCH\ NMe_2$<br>$CH_3$ | 0.1 | 20 | 120 |
| $CH_3$ | | $COCH_2N$ 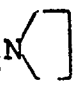 | 0.03 | 30 | 30 |
| $CH_3$ | | $COCH_2N$ 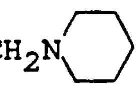 | 1 | 40 | > 120 |
| $CH_3$ | | $COCH_2N$ 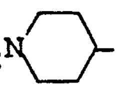 -NHCOPh | 10 | 30 | 30 |
| $CH_3$ | | $COCH_2N$ 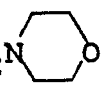 | 0.3 | 48 | 25 |
| $CH_3$ | | $COCH_2N$ 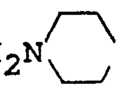 N-$CH_3$ | 0.1 | 49 | > 240 |
| $C_2H_5$ | | $COCH_2N$ 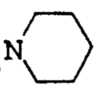 | 3 | 30 | > 120 |
| $CH_3$ | Isomer I | COCH-N 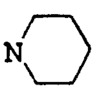<br>$CH_3$ | 3 | 40 | > 150 |
| $CH_3$ | Isomer II | COCH-N 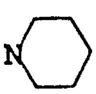<br>$CH_3$ | 3 | 40 | 60 |

2. Blutdruck am Hund:

Methode: Beagle-Hunde beiderlei Geschlechts (Gewicht im Bereich 10 bis 15 kg) wurden mit Natriumpentobarbital narkotisiert (35 mg/kg, i.v.). Nach Einführung eines Endotrachealrohrs wurden sowohl die Oberschenkelarterie als auch die -vene zur Aufnahme des Blutdrucks und zur Verabreichung der Verbindung kanüliert. Der Druck im linken Ventrikel wurde mit einem Katheterspitzen-Messwandler (Millar-Kathether) gemessen und über einen Hugo Sachs-Differentiator differenziert. Sämtliche Methoden wurden auf einem Hellige-Schreibgerät mit sechs Kanälen aufgezeichnet. Die Elektrokardiogrammsignale wurden auf einem Becton-Dickinson-Gerät verfolgt.

Eine einprozentige Lösung der erfindungsgemäßen Verbindung in destilliertem Wasser wurde hergestellt und in verschiedenen Dosierungen intravenös verabreicht. Die bei diesem Modell mit typischen erfindungsgemäßen Verbindungen erhaltenen Resultate sind in der nachfolgenden Tabelle angeführt:

| Verbindung | | Dosis (mg/kg) | Blutdruck-abfall (mm Hg) | Dauer (Min.) | Pulszu-nahme (Puls-schläge pro Min.) | % Zunahme in dp/dt max |
|---|---|---|---|---|---|---|
| 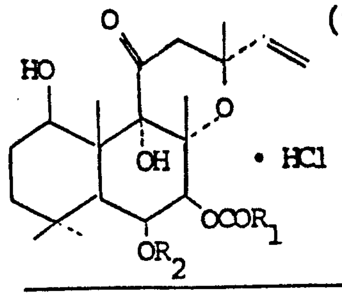 • HCl | | | | | | |
| $R_1$ | $R_2$ | | | | | |
| $CH_3$ | Isomer II $COCH(CH_3)-NMe_2$ | 0.01 | 36 | 22 | - | -36 |
| | | 0.03 | 23 | 40 | +11 | - 4 |
| | | 0.1 | 45 | 60 | +31 | +63 |
| $CH_3$ | $COCH_2N$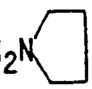 | 0.03 | 14 | 23 | + 9 | +42 |
| | | 0.1 | 49 | 50 | +12 | +35 |
| | | 0.3 | 94 | 90 | +18 | +46 |
| $CH_3$ | $COCH_2N$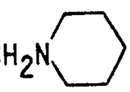 | 0.3 | 15 | 10 | + 4 | + 4 |
| | | 0.1 | 30 | 15 | + 5 | +27 |
| | | 0.3 | 45 | 90 | +14 | +23 |
| $C_2H_5$ | $COCH_2N$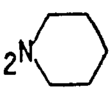 | 0.3 | 10 | 10 | + 9 | +38 |
| | | 1.0 | 46 | 60 | +19 | +65 |
| $CH_3$ | Isomer II $COCH(CH_3)-N$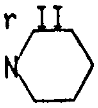 | 0.3 | 13 | 10 | + 5 | +48 |
| | | 1.0 | 80 | 90 | - | -45 |
| $CH_3$ | $COCH_2CH_2N$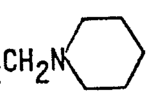 | 0.03 | 4 | 5 | +21 | +60 |
| | | 0.1 | 53 | 150 | +13 | -21 |

Blutdruck an der spontan hypertonen Ratte (Schwanzmanschettenmethode)

Für diesen Versuch wurden spontan hypertone Ratten (Stamm Okamoto) ausgewählt, deren Blutdruck im Bereich zwischen 160 und 240 mm Hg lag. Die Tiere wurden je 20 Minuten lang in eine bei 38°C gehaltene Klimakammer eingebracht. Der Blutdruck wurde an wachen Tieren mit Hilfe eines piezoelektrischen Kristalls in einer um den Schwanz gedrehten Gummimanschette gemessen.

Gruppen von je 6 Tieren erhielten in destilliertem Wasser gelöste erfindungsgemäße Verbindungen 3 Tage lang in einer oralen Tagesdosis von 25 mg/kg, und der Blutdruck wurde 2 Stunden nach der Verabreichung gemessen. Die bei diesem Modell für typische erfindungsgemäße Verbindungen erhaltenen Ergebnisse sind in der nachfolgenden Tabelle angeführt:

| Verbindung | | Dosis (mg/kg) | Abnahme des systolischen Blutdrucks (mm Hg) |
|---|---|---|---|
| 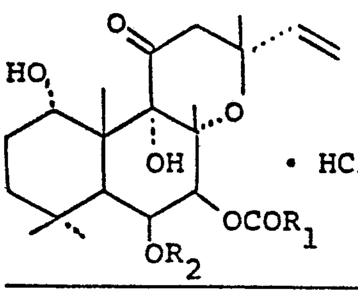 • HCl | | | |
| $R_1$ | $R_2$ | | |
| $CH_3$ | 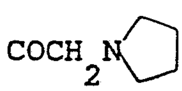 | 25 | 11 |
| $CH_3$ | 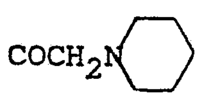 | 25 | 30 |
| $CH_3$ | 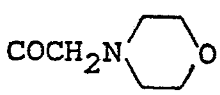 | 25 | 24 |
| $CH_3$ Isomer I | 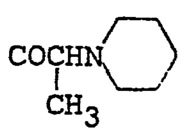 | 25 | 11 |

Die Erfindung wird durch die nachfolgenden Beispiele erläutert, jedoch nicht eingeschränkt:

Beispiel 1

7ß-Acetoxy-1α-t-butyldimethylsilyloxy-6ß,9α-dihydroxy-8,13-epoxy-labd-14-en-11-on (VI, R = t-Butyldimethylsilyl)

Man gibt t-Butylchlordimethylsilan (3,6 g) zu einem Gemisch aus Imidazol (2,52 g) und 7ß-Acetoxy-8,13-epoxy-1α,6ß,9α-trihydroxy-labd-14-en-11-on (6,0 g) in wasserfreiem Dimethylformamid (12 ml), rührt 22 Stunden lang bei 70°C und engt im Vakuum ein. Der Rückstand wird mit Essigester extrahiert und der Extrakt mit Wasser und Kochsalzlösung gewaschen, über wasserfreiem Natriumsulfat getrocknet und eingeengt. Der dabei erhaltene Rückstand wird durch Flash-Säulenchromatographie über Kieselgel unter Verwendung von Essigester : Hexan (3 : 7) als Eluiermittel gereinigt, wobei man 7ß-Acetoxy-1-t-butyldimethylsilyloxy-6ß,9α-dihydroxy 8,13-epoxy-labd-14-en-11-on vom Schmelzpunkt 137 - 139°C in 80 % Ausbeute erhält.

Beispiel 2

1α-t-Butyldimethylsilyloxy-8,13-epoxy-6ß,7ß,9α-trihydroxy-labd-14-en-11-on (IV, R = t-Butyldimethylsilyl)

Man gibt Kaliumcarbonat (2,4 g) zu einer Lösung von 7ß-Acetoxy-1α-t-butyldimethyldisilyloxy-6ß,9α-dihydroxy-8,13-epoxy-labd-14-en-11-on (0,4 g) in einem Gemisch aus Methanol (34 ml) und Wasser (12 ml), rührt 24 Stunden lang bei Raumtemperatur und engt dann im Vakuum ein. Der dabei erhaltene Rückstand wird mit Dichlormethan extrahiert und der Extrakt mit Wasser gewaschen, über wasserfreiem Natriumsulfat getrocknet und der nach Einengen erhaltene Rückstand durch Flash-Säulenchromatographie unter Verwendung von

Chloroform : Diisopropyläther (55 : 45) als Eluiermittel gereinigt, wobei man 1-t-Butyldimethylsilyloxy-8,,13-epoxy-6ß,7ß,9α-trihydroxy-labd-14-en-11-on vom Schmelzpunkt 60 - 62°C in 90 % Ausbeute erhält.

Beispiel 3

1-(t-Butyldimethylsilyloxy)-7ß,9α-dihydroxy-8,13-epoxy-6ß-piperidinoacetoxy-labd-14-en-11-on (II, R = t-Butyldimethylsilyl, $R_3$ und $R_4$ = H, n = O, $NR_5R_6$ = Piperidino)

Man gibt Bromacetylbromid (0,25 ml) unter gutem Rühren zu einer eiskalten Lösung von 1α-t-Butyldimethylsilyloxy-8,13-epoxy-6ß,7ß,9α-trihydroxy-labd-14-en-11-on (1,0 g) und Pyridin (0,31 ml) in Dichlormethan (20 ml) und rührt noch weitere 1,5 Stunden bei 0°C. Dann wäscht man das Reaktionsgemisch mit verdünnter Salzsäure, gesättigter Natriumbicarbonatlösung, Wasser und Kochsalzlösung, trennt die organische Schicht ab, trocknet über wasserfreiem Natriumsulfat und engt im Vakuum ein. Der Rückstand aus 7ß-Bromacetoxy-1-α-t-butyldimethylsilyloxy-6ß,9α-dihydroxy-8,13-epoxy-labd-14-en-11-on (1,2 g) wird mit Piperidin (10 ml) verdünnt und 3 Stunden am Rückfluß erhitzt. Das Reaktionsgemisch engt man im Vakuum ein, extrahiert den Rückstand mit Essigester wäscht die Essigesterschicht mit Wasser, trocknet über wasserfreiem Natriumsulfat und engt ein. Der dabei erhaltene Rückstand wird durch Flash-Säulenchromatographie über Kieselgel unter Verwendung von Essigester : Petroläther : Triäthylamin (45 : 55 : 0,5) gereinigt, wobei man 1α-t-Butyldimethylsilyloxy-7ß-9α-8,13-epoxy-6ß-piperidinoacetoxy-lab-14-en-11-on enthält. Dieses wird in Äther gelöst und mit ätherischer HCl behandelt, um das entsprechende Hydrochlorid vom Schmelzpunkt 200 - 202°C zu enthalten.

Auf entsprechende Weise wurden die folgenden Verbindungen hergestellt:

1α-t-Butyldimethylsilyloxy-7β,9α-dihydroxy-6β[α'-(N,N-dimethylamino-propionyloxy]-8,13-epoxy-labd-14-en-11-on.

1α-t-Butyldimethylsilyloxy-7β,9α-dihydroxy-8,13-epoxy-6β-pyrrolidino acetoxy-labd-14-en-11-on.

6β-4'-Benzoylaminopiperidino acetoxy-1α-t-butyldimethyl silyloxy-7β,9α-dihydroxy-8,13-epoxy-labd-14-en-11-on.

1α-t-Butyldimethylsilyloxy-7β,9α-dihydroxy-8,13-epoxy-6β-morpholino acetoxy labd-14-en-11-on.

1α-t-Butyldimethylsilyloxy-7β,9α-dihydroxy-8,13-epoxy-6β-(N-methylpiperazino acetoxy) labd-14-en-11-on.

1α-t-Butyldimethylsilyloxy-7β-9α-dihydroxy-8,13-epoxy-6β-[α'-piperidinopropionyloxy]-labd-14-en-11-on.

1α-t-Butyldimethylsilyloxy-7β-9α-dihydroxy-8,13-epoxy-6β-[α'-morpholino propionyl]-labd-14-en-11-on.

1α-t-Butyldimethylsilyloxy-7β,9α-dihydroxy-8,13-epoxy-6β-[γ'-piperidino butyryloxy]-labd-14-en-11-on.

Beispiel 4

7ß-Acetoxy-1α-t-butyldimethylsilyloxy-8,13-epoxy-9α-hydroxy-6ß-piperidinoacetoxy-lab-14-en-11-on
(III, R = t-Butyldimethylsilyl, $R_3$ und $R_4$ = H
$NR_5R_6$ = Piperidino)

Man gibt Essigsäure (0,06 ml) zu einem Gemisch aus Dicyclohexylcarbodiimid (0,34 g) und 4-Dimethylaminopyridin (0,11 g) in Essigester (15 ml), rührt 10 Minuten lang bei

Raumtemperatur, versetzt mit 1α-t-Butyldimethylsilyloxy-7ß,9α-dihydroxy-8,13-epoxy-6ß-piperidinoacetoxy-labd-14-e-11-on (0,5 g) und rührt noch über Nacht weiter. Der Überschuß Dicyclohexylcarbodiimid im Reaktionsgemisch wird durch Zusatz von Essigsäure zersetzt und das Reaktionsgemisch mit gesättigter Natriumbicarbonatlösung, Wasser und Kochsalzlösung gewaschen. Die organische Schicht wird über wasserfreiem Natriumsulfat getrocknet und eingeengt. Der dabei erhaltene Rückstand wird durch Flash-Säulenchromatographie unter Verwendung von Essigester : Triäthylamin (45 : 55 : 0,5) als Eluiermittel gereinigt, wobei man 7ß-Acetoxy-1α-t-butyldimethylsilyloxy-8,13-epoxy-9α-hydroxy-6ß-piperidiono-acetoxy-labd-14-en-11-on vom Schmelzpunkt 115° - 117°C in 60 % Ausbeute erhält.

Auf entsprechende Weise wurden die folgenden Verbindungen hergestellt:

7β-Acetoxy-1α-t-butyldimethylsilyloxy-6β[α'-(N,N-dimethylamino)propionyloxy]-8,13-epoxy-9α-hydroxy-labd-14-en-11-on. (I-Isomer).

7β-Acetoxy-1α-t-butyldimethylsilyloxy-6β[α'(N,N-dimethylamino)propionyloxy]-8,13-epoxy-9α-hydroxy-labd-14-en-11-on. (II-Isomer).

7β-Acetoxy-1α-t-butyldimethylsilyloxy-8,13-epoxy-9α-hydroxy-6β-pyrrolidino acetoxy-labd-14-en-11-on.

7β-Acetoxy-6β-[4'-benzoylamino piperidino acetoxy]-1α-t-butyl dimethylsilyloxy-8,13-epoxy-9α-hydroxy-labd-14-en-11-on.

7β-Acetoxy-1α-t-butyldimethylsilyloxy-8,13-epoxy-9α-hydroxy-6β-morpholino acetoxy-labd-14 en-11 on.

7β-Acetoxy-1α-t-butyldimethylsilyloxy-8,13-epoxy-9α-hydroxy-6β-(N-methylpiperazino acetoxy)-labd-14-en-11-on.

1α-t-Butyldimethylsilyloxy-8,13-epoxy-9α-hydroxy-6β-piperidino acetoxy-7β-propionyloxy-labd-14-en-11-on.

7β-Acetoxy-1α-t-butyldimethylsilyloxy-8,13-epoxy-9α-hydroxy 6β[α'-piperidinopropionyloxy-labd-14-en-11-on. (I-Isomer).

7β-Acetoxy-1α-t-butyldimethylsilyloxy-8,13-epoxy-9α-hydroxy-6β[α'-piperidinopropionyloxy]-labd-14-en-11-on. (II-Isomer).

7β-Acetoxy-1α-t-butyldimethylsilyloxy-8,13-epoxy-9α-hydroxy-6β[α'-morpholinopropionyloxy]-labd-14-en-11-on. (I-Isomer).

7β-Acetoxy-1α-t-butyldimethylsilyloxy-8,13-epoxy-9α-hydroxy-6β[α'morpholino propionyloxy]-labd-14-en-11-on. (II-Isomer).

Beispiel 5

7ß-Acetoxy-1α,9α-dihydroxy-8,13-epoxy-6ß-piperidinoacetoxy-lab-14-en-11-on (I, $R_1$ = $CH_3$, $R_2$ = Piperidinoacetyl)

Man rührt 7ß-Acetoxy-1α-butyldimethylsilyloxy-8,13-epoxy-9α-hydroxy-6ß-piperidinoacetoxy-labd-14-en-11-on (1,8 g) in wasserfreiem Tetrahydrofuran (50 ml) mit Tetrabutylammoniumfluorid (4,1 ml; 1 m-Lösung in THF) eine halbe Stunde lang bei Raumtemperatur und engt dann ein, extrahiert den Rückstand mit Essigester und wäscht die organische Schicht mit Wasser und Kochsalzlösung, trocknet über wasserfreiem Natriumsulfat und engt ein. Der dabei erhaltene Rückstand wird durch Flash-Säulenchromatographie unter Verwendung eines Gemischs aus Essigester :

Petroläther : Triäthylamin (45 : 55 : 0,5) als Eluiermittel gereinigt. Umkristallisieren aus Essigester : Petroläther liefert 7-ß-Acetoxy-1α-9α-dihydroxy-8,13-epoxy-6ß-piperidinoacetoxy-labd-14-en-11-on vom Schmelzpunkt 141° - 143°C in 70 % Ausbeute.

Beispiel 6

7ß-Acetoxy-1α,9α-dihydroxy-8,13-epoxy-6ß-piperidinoacetoxy-labd-14-en-11-on-hydrochlorid-hemihydrat
(I, $R_1$= $CH_3$, $R_2$= Piperidonoacetyl)

Man löst 7ß-Acetoxy-1α,9α-dihydroxy-8,13-epoxy-6ß-piperidinoacetoxy-labd-14-en-11-on (0,4 g) in Methanol (2 ml) und behandelt bei 0°C mit ätherischer HCl. Man verdünnt mit wasserfreiem Äther, sammelt das auskristallisierte Hydrochlorid durch Filtrieren, wäscht 7ß-Acetoxy-1α,9α-dihydroxy-8,13-epoxy-6ß-piperidinoacetoxy-labd-14-en-11-on-hydrochlorid-hemiydrat vom Schmelzpunkt 238 - 240°C erhält.

Auf entsprechende Weise wurden die folgenden Verbindungen hergestellt:

7β-Acetoxy-6β-[α'-(N,N-dimethylamino)propionyloxy]-1α,9β-dihydroxy-8,13-epoxy labd-14-en-11-on hydrochlorid (I-Isomer).
Fp. 252-255°C.

7β-Acetoxy-6β-[α'-(N,N-dimethylamino)propionyloxy]-1α,9α-dihydroxy-8,13-epoxy labd-14-en-11-on hydrochlorid hemihydrat (II-Isomer).
Fp. 215°-217°C.

7β-Acetoxy-1α,9α-dihydroxy-8,13-epoxy-6β-pyrrolidinoacetoxy labd-14-en-11-on hydrochlorid.
Fp. 249°C.

7β-Acetoxy-1α,9α-dihydroxy-8,13-epoxy-6β-piperidino acetoxy labd-14-en-11-on hydrochloride hemihydrat.
Fp. 238-240°C.

7β-Acetoxy-6β-[4'-benzoylaminopiperidino acetoxy]-1α,9α-dihydroxy-8,13-epoxy labd-14-en-11-on hydrochloride dihydrat.
Fp. 104-106°C.

7β-Acetoxy-1α,9α-dihydroxy-8,13-epoxy-6β-morpholino acetoxy labd-14-en-11-on hydrochloride hydrat.
Fp. 173-75°C.

7β-Acetoxy-1α,9α-dihydroxy-8,13-epoxy-6β-(N-methyl piperazino acetoxy)labd-14-en-11-on dihydrochloride hydrat.
Fp. 180-186°C.

1α,9α-Dihydroxy-8,13-epoxy-6β-piperidino acetoxy-7β-propionyloxy-labd-14-en-11-on hydrochlorid, F.p. 230-231°C.

7β-Acetoxy-1α,9α-dihydroxy-8,13-epoxy-6β-[α'-piperidino propionyloxy]-labd 14-en-11-on hydrochlorid.
(I-Isomer).
Fp. 245-247°C.

7β-Acetoxy-1α,9α-dihydroxy-8,13-epoxy-6β-[α'-piperidino propionyloxy]-labd-14-en-11-on hydrochlorid.
(II-Isomer).
Fp. 268-269°C.

7β-Acetoxy-1α,9α-dihydroxy-8,13-epoxy-6β-[α'-morpholino propionyloxy]-labd-14-en-11-on hydrochloride monohydrat
(I-Isomer).
Fp. 178-180°C.

7β-Acetoxy-1α,9α-dihydroxy-8,13-epoxy-6β-[α'-morpholino propionyloxy]-labd-14-en-11-on hydrochloride trihydrat (II-Isomer).
Fp. 190-198°C.

7β-Acetoxy-1α,9α-dihydroxy-8,13-epoxy-6β-[α'-piperidino propionyloxy]-labd-14-en-11-on hydrochlorid.
Fp. 206-210°C.

7β-Acetoxy-1α,9α-dihydroxy-8,13-epoxy-6β-[α'-morpholino propionyloxy]-labd-14-en-11-on hydrochlorid.
Fp. 198-200°C.

7β-Acetoxy-1α,9α-dihydroxy-8,13-epoxy-6β-[γ'-piperidino butyryloxy]-labd-14-en-11-on hydrochlorid hydrat.
Fp. 81-83°C.

Beispiel 7

7ß-Acetoxy-6ß,9α-dihydroxy-8,13-epoxy-1α-[2'-methoxyäthoxy)-methoxy]-labd-14-en-11-on (VI, R= (2'-Methoxyäthoxy)-methyl)

Man gibt 7ß-Acetoxy-8,13-epoxy-1α,6ß-trihydroxy-labd-14-en-11-on (5,0 g) zu einem Gemisch aus frisch destilliertem Methoxyäthoxymethylchlorid (3,04 ml) und Triäthylamin (3 bis 4 ml) in wasserfreiem Acetonitril (100 ml). Man rührt das Reaktionsgemisch eine halbe Stunde lang, erhitzt dann 12 Stunden lang am Rückfluß, engt dann im Vakuum ein und extrahiert den Rückstand mit Essigester. Die organische Schicht wird abgetrennt, mit Wasser und Kochsalzlösung gewaschen, über wasserfreiem Natriumsulfat getrocknet und eingeengt. Der dabei erhaltene Rückstand wird durch Flash-Säulenchromatographie unter Verwendung von Chloroform : Diisopropyläther (4 : 6) als Eluiermittel gereinigt, wobei man 7ß-Acetoy-6ß-9α-dihydroxy-8,13-epoxy-1α-[(2'-methoxäthoxy)-methoxy]-labd-14-en-11-on vom Schmelzpunkt 128°C in 70 % Ausbeute erhält.

Beispiel 8

8,13-Epoxy-1α-[(2'-methoxyäthoxy)-methoxy]-6ß,7ß,9α-trihydroxy-labd-14-en-11-on (IV, R= (2'-Methoxyäthoxy)-methyl)

Man rührt ein Gemisch aus 7ß-Acetoxy-6ß,9α-dihydroxy-8,13-epoxy-1α-[(2'-methoxyäthoxy)-methoxy]-labd-14-en-11-on (12,5 g), Methanol (160 ml) und wäßriger Natronlauge (40 ml, 2,75 %ig) zwei Stunden lang bei Raumtemperatur, engt im Vakuum ein und extrahiert den Rückstand mit Essigester. Die organische Schicht wird mit Wasser und Kochsalzlösung gewaschen, über wasserfreiem Natriumsulfat getrocknet und eingeengt. Der Rückstand wird durch Flash-Säulenchromatographie unter Verwendung von Acetonitril : Diisopropyläther (2,5 : 97,5) als Eluiermittel gereinigt, wobei man 8,13-Epoxy-1α-[(2'-methoxyäthoxy)-methoxy]-6ß,7ß,9α-trihydroxy-labd-14-en-11-on als hochviskoses Öl in 80 % Ausbeute erhält.

Beispiel 9

7ß,9α-Dihydroxy-8,13-epoxy-1α-[(2'-methoxyäthoxy)-methoxy]-6ß-piperidinoacetoxy-labd-14-en-11-on (II, R= (2'-Methoxyäthoxy)-methyl, $R_3$ und $R_4$= H, $NR_5R_6$= Piperidino)

Man gibt Pyridin (0,9 ml) zu einem im Eis/Salzbad gekühlten Gemisch aus 8,13-Epoxy-1-[(2'-methoxyäthoxy)-methoxy]-6ß,7ß,9α-trihydroxy-labd-14-en-11-on 4,56 g), Chloracetylchlorid (0,95 ml) und wasserfreiem Dichlormethan (20 ml), rührt dann drei Stunden lang bei 0°C und danach 16 Stunden lang bei Raumtemperatur, wäscht dann mit Wasser und Kochsalzlösung, trocknet die organische Schicht über wasserfreiem Natriumsulfat und engt ein. Der durch gründliches Trocknen im Hochvakuum erhaltene Rückstand wird in trockenem Piperidin (20 ml) aufgelöst und drei Stunden

lang am Rückfluß erhitzt. Das Reaktionsgemisch engt man im Vakuum ein, extrahiert den Rückstand mit Essigester, wäscht die Essigesterschicht mit Wasser, trocknet sie über wasserfreiem Natriumsulfat und engt ein. Der dabei erhaltene Rückstand wird durch Flash-Säulenchromatographie unter Verwendung von Essigester : Petroläther : Triäthylamin (40 : 60 : 0,5) als Eluiermittel gereingt, wobei man 7ß,9α-Dihydroxy-8,13-epoxy-1α-[2'-methoxyäthoxy-methoxy]-6ß-piperidinoacetoxy-labd-14-en-11-on erhält. Dieses löst man in Methanol und behandelt mit ätherischem HCl, um das entsprechende Hydrochlorid vom Schmelzpunkt 203 - 205°C zu erhalten.

Beispiel 10

7ß-Acetoxy-8,13-epoxy-9α-hydroxy-1α-[(2'-methoxyäthoxy)-methoxy]-6ß-piperidinoacetoxy-labd-14-en-11-on (III, R= (2'-Methoxyäthoxy)-methyl, $R_3$ und $R_4$= H, $NR_5R_6$= Piperidino)

Man verfährt wie in Beispiel 4. Umsetzung von Essigsäure mit 7ß-9α-Dihydroxy-8,13-epoxy-1α- (2'-methoxyäthoxy)-methoxy - 6ß-piperidinoacetoxy-labd-14-en-11-on in Gegenwart von Dicyclohexylcarbodiimid und Dimethylaminopyridin ergibt 7ß-Acetoxy-8,13-epoxy-9α-hydroxy-1α-[(2'-methoxyäthoxy)-methoxy]-6ß-piperidinoacetoy-labd-14-en-11-on in 80 % Ausbeute; Schmelzpunkt 113 - 114°C nach Umkristallisieren aus Petroläther (40° bis 60°C).

Beispiel 11

7β-Acryloyloxy-1-t-butyldimethylsilyloxy-6β,9α-dihydroxy-8,13-epoxy-labd-14-en-11-on

Acrylsäure (2,28 ml, 33,2 mMol) wird zu einer Mischung von DCC (Dicyclohexylcarbodiimid) (16,56 g, 80,38 mMol) und 4-Dimethylaminopyridin (2,02 g, 16,56 mMol) in Essigester (150 ml) gegeben und die Mischung 10 Minuten bei Raumtemperatur gerührt. Man fügt 1α-t-Butyldimethylsilyloxy-8,13-epoxy-6β,7β,9α-trihydroxylabd-14-en-11-on (8,0 g, 16,1 mMol) zu der Mischung und setzt das Rühren über Nacht fort. Ein Überschuß von DCC in dem Reaktionsgemisch wird zersetzt durch Zugabe von Essigsäure, anschließend wird die Mischung filtriert. Das Filtrat wird mit gesättigter Natriumbicarbonatlösung, anschließend mit Wasser und Kochsalzlösung gewaschen. Die organische Schicht wird über wasserfreiem Natriumsulfat getrocknet und eingedampft. Der Rückstand wird durch flash-Säulenchromatographie unter Benutzung von Essigsäurethylester/Petrolether (1 : 3) als Laufmittel gereinigt. Ausbeute 40 %. Die auf diese Weise erhaltene Verbindung wird ohne Kristallisation für die nächste Verfahrensstufe benutzt.

Beispiel 12

6β-Acryloyloxy-1-t-butyldimethylsilyloxy-7β,9α-dihydroxy-8,13-epoxy-labd-14-en-11-on

Zu der gerührten Lösung von 7β-Acryloyloxy-1-t-butyldimethylsilyloxy-6β,9α-dihydroxy-8,13-epoxy-labd-14-en-11-on (2,4 g) in Acetonitril/Wasser (2 : 1, 120 ml) wird eine Natriumhydroxid-Lösung (1 N, 8,4 ml) bei Raumtemperatur hinzugegeben. Das Rühren wird über 25 min. fortgesetzt, die Reaktionsmischung im Vakuum eingedampft

und mit Essigsäureethylester extrahiert. Die organische Schicht wird mit Wasser gewaschen und anschließend über wasserfreiem Natriumsulfat getrocknet und eingeengt. Der Rückstand wird chromatographiert unter Benutzung von flash-Säulenchromatographie und dem Laufmittel Essigsäureethylester/Petrolether (9 : 41) als Laufmittel; Ausbeute 65 %, Schmelzpunkt 164 - 166°C.

Beispiel 13

7β-Acetoxy-6β-acryloyloxy-1-t-butyl-dimethylsilyl-8,13-epoxy-9α-hydroxy-labd-14-en-11-on

Man verfährt wie in Beispiel 3. Ausgehend von 6β-Acryloyloxy-1-t-butyldimethylsilyloxy-7β,9α-dihydroxy-8,13-epoxy-labd-14-en-11-on wird die Verbindung 7β-Acetoxy-6β-acryloyloxy-1-t-butyldimethylsilyl-8,13-epoxy-9α-hydroxy-labd-14-en-11-on in 80 %iger Ausbeute erhalten. Die Verbindung wird für den nächsten Verfahrensschritt ohne Kristallisation benutzt.

Beispiel 14

7β-Acetoxy-1α,9α-dihydroxy-8,13-epoxy-6β-(β'-piperidino-propionyloxy)labd-14-en-11-on

Man gibt trockenes Piperidin (1 ml) zu der gerührten Lösung von 7β-Acetoxy-6β-acryloyloxy-1-t-butyldimethylsilyloxy-8,13-epoxy-9α-hydroxy-labd-14-en-11-on (0,32 g, 0,51 mMol) in trockenem Ether (10 ml). Die Reaktionsmischung wird eine weitere Stunde bei Raumtemperatur gerührt und dann im Hochvakuum eingeengt. Das Rohprodukt wird mit Tetrabutylammoniumfluorid (1 M-Lösung in THF 0,61 ml, 0,61 mMol) behandelt wie in Beispiel 5 beschrieben. Die Verbindung wird gereinigt durch flash-Säulenchromatographie unter Benutzung von Essigsäureethylether/Petrolether (4 : 6) als Laufmittel; Ausbeute 83,8 %, Schmelzpunkt 93 - 96°C.

Entsprechend wird die folgende Verbindung hergestellt:

7β-Acetoxy-1α,9α-dihydroxy-8,13-epoxy-6β(β'-morpholino-propionyloxy)-labd-14-en-11-on (isoliert als Hydrochlorid).

Patentansprüche:

1. Verbindungen der Formel

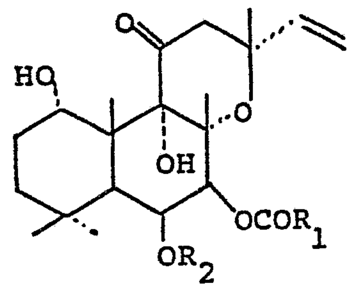

I

worin bedeuten:

$R_1$ eine geradkettige oder verzweigte, unsubstituierte oder ein- oder mehrfach durch Hydroxy oder Halogen substituierte $C_1$-$C_{20}$-Alkylgruppe

$R_2$ den Rest

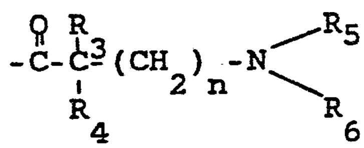

wobei $R_3$ und $R_4$ gleich oder verschieden sein können und für Wasserstoff oder Alkyl stehen,

n eine ganze Zahl von 0 bis 10 bedeutet, und

$R_6$ Wasserstoff bedeutet und $R_5$ für Wasserstoff, unsubstituiertes oder substituiertes Alkyl, Cycloalkyl, Aralkyl, Aryl, einen Heterocyclus, Amino, substituiertes Amino, Hydroxy, Acyl, Dialkylaminoalkyl, Carbamyl, Carboxyalkyl oder Carbalkoxyalkyl steht, oder

$R_5$ und $R_6$ gleich sind und unsubstutiertes oder substituiertes Alkyl, Aryl oder Aralkyl bedeuten, oder $R_5$ eine Alkylgruppe bedeutet und $R_6$ für substituiertes Alkyl, Cycloalkyl, Aralkyl oder Dialkylaminoalkyl steht, oder

$R_5$ und $R_6$ zusammen mit dem Stickstoffatom, an das sie gebunden sind, für einen Heterocyclus stehen, der

zusätzlich zum N-Atom ein oder mehrere Heteroatome enthalten kann und ein- oder mehrfach mit Alkyl, Aralkyl, Hydroxyalkyl, Aryl, Hydroxyl oder weitere heterocyclische Reste substituiert sein kann, sowie deren physiologisch unbedenkliche Salze.

2. Verbindungen der Formel I gemäß Anspruch 1, worin $R_1$ eine unsubstituierte $C_1$-$C_6$-Alkylgruppe, oder eine $C_1$-$C_6$-Alkylgruppe, die mit Methyl, Hydroxymethyl oder Halogenmethyl substituiert ist, bedeutet.

3. Verbindungen gemäß Anspruch 1, worin $R_1$ eine unsubstituierte oder mit Methyl, Hydroxymethyl oder Halogenmethyl substituierte $C_1$-$C_6$-Alkyl-Gruppe, und $R_2$ den Rest

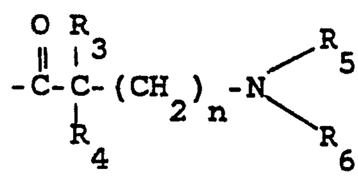

darstellt, worin $R_3$ und $R_4$ Wasserstoff bedeuten, n eine ganze Zahl von 0 bis 5 darstellt und $R_5$ und $R_6$ entweder gleich oder verschieden sind und $C_1$-$C_6$-Alkyl bedeuten oder zusammen mit dem N-Atom, an das sie gebunden sind, einen unsubstituierten oder substituierten Piperidino-Pyrrolidino-, Morpholino-, Piperazino-, Thiomorpholino-, Imidazolino- oder Theophyllin-Rest darstellen.

4. Verbindungen gemäß Anspruch 1, worin $R_1$ eine unsubstituierte oder mit Methyl, Hydroxymethyl oder Halogenmethyl substituierte $C_1$-$C_6$-Alkyl-Gruppe, und $R_2$ den Rest

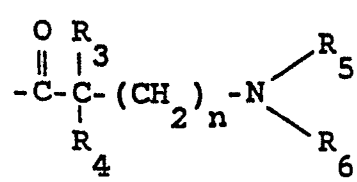

darstellt, worin
$R_3$ Wasserstoff, $R_4$ eine $C_1$-$C_6$-Alkyl oder Arylgruppe bedeutet, n eine ganze Zahl von 0 bis 5 darstellt und $R_5$ und $R_6$ die genannten Bedeutungen haben.

5. Verbindungen gemäß Anspruch 4, worin $R_5$ und $R_6$ entweder gleich oder verschieden sind und $C_1$-$C_6$-Alkyl bedeuten oder zusammen mit dem N-Atom, an das sie gebunden sind, den Piperidino-, Pyrrolidino-, Morpholino-, Piperazino-, Thiomorpholino-, Imidazolino- oder Theophyllin-Rest darstellen.

6. Verfahren zur Herstellung von Verbindungen gemäß Anspruch 1, dadurch gekennzeichnet, daß man eine Verbindung der Formel II

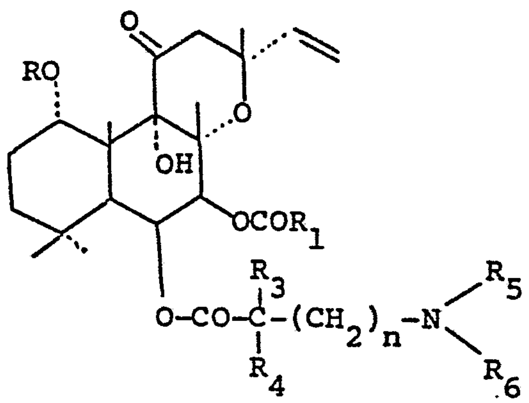

woirn $R_3$, $R_4$, $R_5$, $R_6$ und n die genannten Bedeutungen haben und R eine Schutzgruppe für eine alkoholische Hydroxygruppe bedeutet, nach üblichen Mehoden acyliert zu einer Verbindung der Formel III

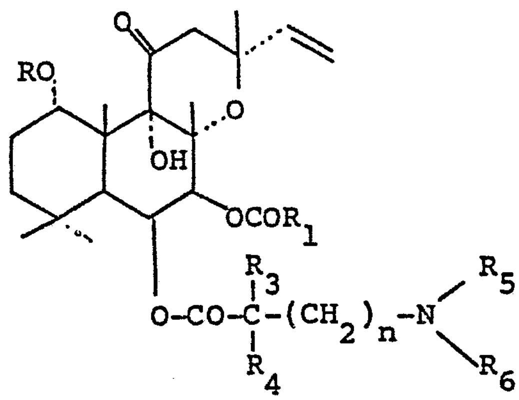

III

worin $R_1$ die genannte Bedeutung hat, und schließlich in üblicher Weise die Schutzgruppe in 1-Stellung abspaltet.

7. Verfahren gemäß Anspruch 6, dadurch gekennzeichnet, daß R die Trimethylsilyl-, Methoxyäthoxymethyl- oder Methoxymethylgruppe bedeutet.

8. Arzneimittel, gekennzeichnet durch einen Gehalt an einer Verbindung der Formel I gemäß Anspruch 1 oder eines physiologisch unbedenklichen Salzes davon.

9. Verwendung einer Verbindung gemäß Anspruch 1 zur Herstellung eines Arzneimittels mit blutdrucksenkender Wirkung.

<u>Patentansprüche Österreich und Spanien:</u> **0252482**

1. Verfahren zur Herstellung von Verbindungen der Formel I

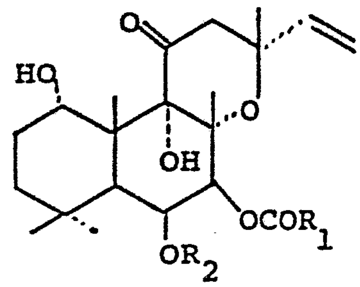

I

worin bedeuten:

$R_1$ eine geradkettige oder verzweigte, unsubstituierte oder ein- oder mehrfach durch Hydroxy oder Halogen substituierte $C_1$-$C_{20}$-Alkylgruppe

$R_2$ den Rest

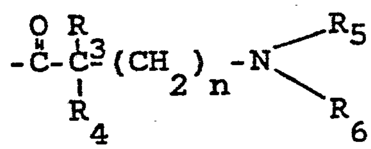

wobei $R_3$ und $R_4$ gleich oder verschieden sein können und für Wasserstoff oder Alkyl stehen,
n eine ganze Zahl von 0 bis 10 bedeutet, und
$R_6$ Wasserstoff bedeutet und $R_5$ für Wasserstoff, unsubstituiertes oder substituiertes Alkyl, Cycloalkyl, Aralkyl, Aryl, einen Heterocyclus, Amino, substituiertes Amino, Hydroxy, Acyl, Dialkylaminoalkyl, Carbamyl, Carboxyalkyl oder Carbalkoxyalkyl steht, oder
$R_5$ und $R_6$ gleich sind und unsubstutiertes oder substituiertes Alkyl, Aryl oder Aralkyl bedeuten, oder $R_5$ eine Alkylgruppe bedeutet und $R_6$ für substituiertes Alkyl, Cycloalkyl, Aralkyl oder Dialkylaminoalkyl steht, oder
$R_5$ und $R_6$ zusammen mit dem Stickstoffatom, an das sie gebunden sind, für einen Heterocyclus stehen, der

zusätzlich zum N-Atom ein oder mehrere Heteroatome enthalten kann und ein- oder mehrfach mit Alkyl, Aralkyl, Hydroxyalkyl, Aryl, Hydroxyl oder weitere heterocyclische Reste substituiert sein kann, sowie deren physiologisch unbedenkliche Salze, dadurch gekennzeichnet, daß man eine Verbindung der Formel II

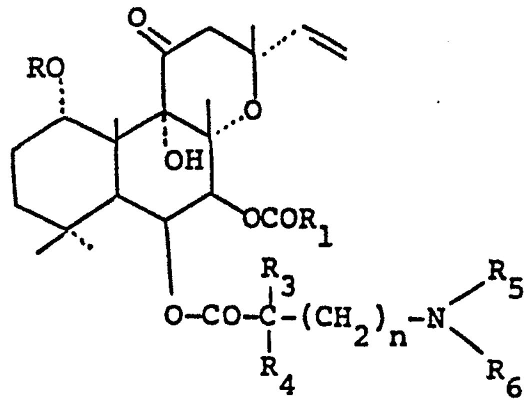

worin $R_3$, $R_4$, $R_5$, $R_6$ und n die genannten Bedeutungen haben und R eine Schutzgruppe für eine alkoholische Hydroxygruppe bedeutet, nach üblichen Mehoden acyliert zu einer Verbindung der Formel III

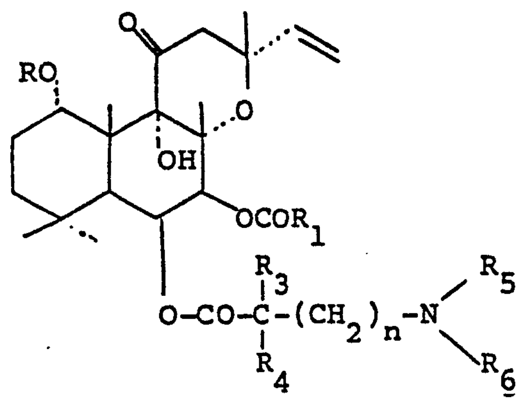

III

worin $R_1$ die genannte Bedeutung hat und schließlich in üblicher Weise die Schutzgruppe in 1-Stellung abspaltet.

2. Verfahren gemäß Anspruch 1, dadurch gekennzeichnet, daß Verbindungen der Formel I hergestellt wurden, worin $R_1$ eine unsubstituierte $C_1$-$C_6$-Alkylgruppe oder eine $C_1$-$C_6$-Alkylgruppe, die mit Methyl, Hydroxymethyl oder Halogenmethyl substituiert ist, bedeutet, und $R_2$ den Rest

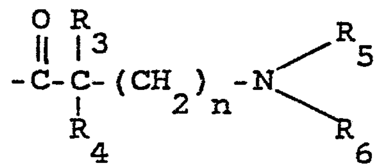

darstellt, worin $R_3$ und $R_4$ Wasserstoff bedeuten, n eine ganze Zahl von 0 bis 5 darstellt und $R_5$ und $R_6$ entweder gleich oder verschieden sind und $C_1$-$C_6$-Alkyl bedeuten oder zusammen mit dem N-Atom, an das sie gebunden sind, einen unsubstituierten oder substituierten Piperidino-Pyrrolidino-, Morpholino-, Piperazino-, Thiomorpholino-, Imidazolino- oder Theophyllin-Rest darstellen.

3. Verfahren gemäß Anspruch 1, dadurch gekennzeichnet, daß Verbindungen der Formel I hergestellt werden, worin $R_1$ eine unsubstituierte oder mit Methyl, Hydroxymethyl oder Halogenmethyl substituierte $C_1$-$C_6$-Alkylgruppe bedeutet und $R_2$ den Rest

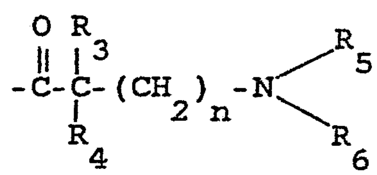

darstellt, worin $R_3$ Wasserstoff, $R_4$ eine $C_1$-$C_6$-Alkyl- oder Arylgruppe bedeutet, n eine ganze Zahl von 0 bis 5 darstellt und $R_5$ und $R_6$ die genannten Bedeutungen haben.

4. Verfahren gemäß Anspruch 3, dadurch gekennzeichnet, daß Verbindungen der Formel I hergestellt werden, worin $R_1$, $R_2$, $R_3$ und $R_4$ und n die genannten Bedeutungen haben

und $R_5$ und $R_6$ zusammen mit dem N-Atom, an das sie gebunden sind, den Piperidino-, Pyrrolidino-, Morpholino-, Piperazino-, Thiomorpholino-, Imidazolino- oder Theophyllin-Rest darstellen.

5. Verfahren gemäß den Ansprüchen 1 - 4, dadurch gekennzeichnet, daß R die Trimethylsilyl-, Methoxyethoxymethyl- oder Methoxymethylgruppe bedeutet.

6 Arzneimittel, gekennzeichnet durch einen Gehalt an einer Verbindung der Formel I gemäß Anspruch 1 oder eines physiologisch unbedenklichen Salzes davon.

7. Verwendung einer Verbindung gemäß Anspruch 1 zur Herstellung eines Arzneimittels mit blutdrucksenkender Wirkung.